# EUROPEAN PATENT APPLICATION

(11) **EP 4 098 230 A2**
(43) Date of publication of application: **07.12.2022**
(21) Application number: 22172760.5
(22) Date of filing: 11.05.2022
(51) Int. Cl.: A61F 2/66, A61F 2/60, A61F 2/50, A61F 2/76

(54) **SHOCK ABSORBER SYSTEM FOR A PROSTHESIS**

(30) Priority: 14.05.2021 US 202163188859 P; 14.04.2022 US 202217720999
(71) Applicant: WillowWood Global LLC, Mt. Sterling, OH 43143 (US)
(72) Inventor: Ficanha, Evandro Maicon, Grove City, 43123 (US); Wernke, Matthew M., Tampa, 33647 (US); Tollett, Anne Marie, Grove City, 43123 (US)
(74) Representative: Feder Walter Ebert

(57) **Abstract**

In embodiments, a shock absorber system for a prosthesis includes an outer housing having a bore and attachable to a prosthetic limb; an inner housing, attachable to a prosthetic socket, within the bore for axial and rotational movement relative to the outer housing; a first resilient element within the outer housing that resists axial movement of the inner housing into the bore and urges the inner housing back to an uncompressed configuration; and alternatively or in addition a second resilient element within the outer housing that resists rotational movement of the inner housing relative to the outer housing, wherein a torsional force urging relative rotation between the inner housing and the outer housing causes compression of the second resilient element such that the second resilient element resists the torsional force and urges the inner housing and outer housing back to an aligned configuration.

## Description

The present disclosure relates to prosthetic devices and more particularly, to shock absorber systems that interconnect a prosthetic limb with a prosthetic socket.

Prosthetic devices typically include a socket assembly in which a socket is shaped to fit over and engage a residual limb. The socket assembly includes a connector, such as a female pyramid connector, that engages a male pyramid connector attached to an intermediate device, such as a swivel or pivot that is incorporated in or attached to a prosthetic limb, hand, or foot.

Prosthetic devices such as prosthetic legs or feet must bear a portion of the body weight of the user. During ambulatory activities, which include walking, running, and jumping, the prosthetic device may intermittently bear loads greater than the body weight of the user. To minimize the shock resulting from the impact of the contact of the prosthetic foot or limb on a surface, especially hard and unyielding surfaces, prosthetic legs or feet incorporate structure that absorbs the shock of such impact. Some prosthetic devices include prosthetic feet having components that act as leaf springs that deflect during ambulatory activities to add a measure of resiliency to the prosthetic device. However, a disadvantage of such structures when used alone to absorb shock is that there is no adjustment in effective spring constant to optimize the resiliency of the prosthetic foot for both low impact ambulatory activity such as walking and high impact ambulatory activity such as running and jumping.

Other types of shock absorbing structure have been incorporated into prosthetic limbs such as legs and feet to lessen the shock impact from ambulatory activities to provide a comfortable user experience. For example, an intermediate device interconnecting the prosthetic socket with, for example, a prosthetic foot has been fitted with a spring, such as a coil spring to absorb impact shock. In other examples, the intermediate device is fitted with a spring and a damper, such as a hydraulic cylinder damper, in an attempt to absorb impact shock and minimize spring bounce.

A disadvantage of current prosthetic devices is that they cannot provide optimal shock absorption, which is a function of the effective spring constant of the resilient element of the device, for both low impact and high impact ambulatory activities. If the effective spring constant of the shock absorbing component of the prosthesis is relatively low, which is suitable for low impact ambulatory activities such as walking, the shock absorbing component may bottom out and become ineffective for relatively high impact activities such as running or jumping. Conversely, a shock absorbing component having a relatively high effective spring constant suitable for running or jumping may be too stiff for effective shock absorption for walking.

It is also advantageous to provide a prosthetic device such as a prosthetic leg or foot with the capability to accommodate torsional forces. Such torsional forces may be encountered in ambulatory motion on uneven surfaces or in activities in which the wearer plants their foot and rotates about it. It is also advantageous to provide such a prosthetic device with an alignment capability so that the prosthetic device, such as a foot, aligns itself once the torsional force is removed. Such application and removal of torsional forces may be encountered when the wearer walks on uneven surfaces, turns while walking, or moves laterally.

Accordingly, there is a need for a shock absorber system for a prosthesis that provides an optimal shock absorbing function for both low impact and high impact ambulatory activities of a user. There also is a need for a shock absorbing system for a prosthesis that provides flexibility in response to torsional stresses that are encountered in ambulatory activities of a user, such as the user pivoting on a prosthetic foot.

The present disclosure describes a shock absorber system for a prosthesis that overcomes the disadvantages inherent in shock absorber system components currently used in prosthetic devices. The disclosed shock absorber system is particularly useful for absorbing impact shock over a wide range of compressive forces encountered in ambulatory activities when connected to a prosthetic leg or foot. In embodiments, the disclosed shock absorber system includes multiple resilient elements that cooperate to provide an effective spring constant that varies proportionately in response to the level of stress resulting from the ambulatory activity. In embodiments, the disclosed shock absorber system includes multiple resilient elements that resist torsional stresses and absorb shock loads encountered by prosthetics used for ambulatory activities and perform an alignment function when recovering from relative rotation of the prosthetic limb from such torsional stresses.

In one aspect, a shock absorber system for a prosthesis includes an outer housing having a bore; an inner housing received within the bore for slidable movement of the inner housing relative to the outer housing; an upper resilient element received within the inner housing that resists movement of the inner housing into the bore; and a lower resilient element received within the housing that resists movement of the inner housing into the bore. A compressive force urging the inner housing to move relative to the outer housing from an unstressed configuration into the bore causes compression of the upper resilient element and the lower resilient element such that the upper resilient element and the lower resilient resist the compressive force and urge the inner housing back to the unstressed configuration.

In another aspect, a shock absorber system for a prosthesis includes an outer housing attachable to a prosthetic foot or limb and an inner housing attachable to a prosthetic socket. The outer housing has a cylindrical bore with an upper, proximal opening and a bottom. The inner housing is received within the bore for slidable movement along a central axis of the bore. The inner housing has a cylindrical wall defining a recess with an upper, proximal end, an open lower, distal end, and a distal fitting including a pyramid connector that attaches to a prosthetic socket assembly. A first cylindrical resilient element is received within the recess and resists movement of the inner housing into the bore. A second cylindrical resilient element is received within the recess and resists movement of the inner housing into the bore. An end travel limiter is positioned in the recess and abuts the upper end and an upper end of the first cylindrical resilient element. A central travel limiter is positioned in the recess and abuts a lower end of the first cylindrical resilient element and an upper end of the second cylindrical resilient element, thereby separating the first cylindrical resilient element from the second cylindrical resilient element.

The first cylindrical resilient element is captured between and abuts the end travel limiter and the central travel limiter, and the lower resilient element is captured between and abuts the central travel limiter and the bottom of the cylindrical bore. One or both of the end traveler limiter and the central travel limiter include a boss extending at least partially through a central passage of the first cylindrical resilient element. Engagement of the end traveler limiter and the central travel limiter defines a limit of compression of the first cylindrical resilient element from displacement of the inner housing into the cylindrical bore.

A high impact travel limiter abuts the bottom of the cylindrical bore and is positioned to engage the open lower end of the cylindrical wall of the inner housing, thereby defining a limit of compression of the second cylindrical resilient element from displacement of the inner housing into the cylindrical bore. A compressive force urging the inner housing to move relative to the outer housing from an unstressed configuration into the bore causes compression of the upper resilient element and the lower resilient element such that the upper resilient element and the lower resilient resist the compressive force and urge the inner housing back to the unstressed configuration.

In yet another aspect, a method of making a shock absorber system for a prosthesis to interconnect a prosthetic foot or limb with a prosthetic socket includes forming a bore in an outer housing and forming a recess in an inner housing. The inner housing is placed within the bore for slidable movement of the inner housing relative to the outer housing. An upper resilient element and a lower resilient element are placed within the recess of the inner housing to resist movement of the inner housing into the bore. A central travel limiter is placed in the recess to separate the upper resilient element and the lower resilient element and to provide a limit of compression of the upper resilient element. A limit of travel of the inner housing into the bore is selected whereby a compressive force urging the inner housing to move relative to the outer housing from an unstressed configuration into the bore causes compression of the upper resilient element and the lower resilient element such that the upper resilient element and the lower resilient resist the compressive force and urge the inner housing back to the unstressed configuration.

In another aspect, a shock absorber system for a prosthesis includes an outer housing having a bore, an inner housing received within the bore for relative rotational movement between the inner housing and the outer housing, and a resilient element received within the outer housing that resists the rotational movement. A torsional force that urges the rotational movement from an aligned or unrotated configuration of the inner housing and the outer housing causes compression of the resilient element such that the resilient element resists the torsional force and urges the inner housing and outer housing back to the aligned configuration.

In another aspect, a shock absorber system for a prosthesis includes an outer housing having a bore; an inner housing received within the bore for axial and rotational movement relative to the outer housing; and a first resilient element received within the outer housing that resists the axial movement of the inner housing into the bore, wherein a compressive force that urges the inner housing to move from an uncompressed configuration further into the bore causes compression of the first resilient element such that the first resilient element resists the compressive force and urges the inner housing back to the uncompressed configuration.

The shock absorber system of this aspect also includes a second resilient element received within the outer housing that resists the rotational movement of the inner housing relative to the outer housing. A torsional force that urges relative rotation between the inner housing and the outer housing from an aligned or unrotated configuration of the inner housing and the outer housing causes compression of the second resilient element. The second resilient element resists the torsional force and urges the inner housing and outer housing back to the aligned configuration.

In another aspect of the shock absorber system for a prosthesis, the system includes an outer housing having a bore with a longitudinal axis, a protrusion extending radially into the bore, and an annular ledge in the bore. An inner housing is received within the bore for linear movement along the longitudinal axis and relative rotational movement about the central longitudinal axis between the inner housing and the outer housing. The inner housing has an elongate slot extending in a direction of the central longitudinal axis. A clip is positioned in the bore between the inner housing and the outer housing and includes a rib engaging the elongate slot such that the inner housing slides relative to the clip during the linear movement and the clip and the inner housing rotate in unison during the rotational movement. The clip engages the outer housing to restrict linear movement along the longitudinal axis.

A cap is mounted in the bore and is attached to the outer housing, the cap abutting the clip and the protrusion such that the clip is captured between the cap and the ledge so that movement of the clip along the longitudinal axis is restricted. A first resilient element including an upper resilient member and a lower resilient member is received within the inner housing. The first resilient element resists movement of the inner housing along the longitudinal axis into the bore. A compressive force that urges the inner housing to move from an uncompressed configuration further into the bore to a compressed configuration causes compression of the first resilient element between the inner housing and the outer housing such that the first resilient element resists the compressive force and urges the inner housing back to the uncompressed configuration.

A second resilient element including a first resilient member and a second resilient member is mounted in the bore between the inner housing and the outer housing. The first resilient member and the second resilient member extend between the clip and the protrusion on opposite sides of the inner housing and the first resilient member and the second resilient member are constrained from movement along the longitudinal axis by the ledge and the cap. The first resilient member and the second resilient member resist the relative rotational movement between the inner housing and the outer housing. A torsional force that urges the relative rotation from an aligned or unrotated configuration of the inner housing and the outer housing causes compression of one of the first resilient member and the second resilient member such that the second resilient element resists the torsional force and urges the inner housing and outer housing back to the aligned configuration.

In another aspect, a method of making a shock absorber system for a prosthesis to interconnect a prosthetic foot or limb with a prosthetic socket includes forming a bore in an outer housing and forming a protrusion extending into the bore; forming a recess in an inner housing; and placing the inner housing within the bore for axial and rotational movement relative to the outer housing. A first resilient element is placed within the recess of the inner housing, the first resilient element including an upper resilient member and a lower resilient member within the housing to resist movement of the inner housing into the bore. A central travel limiter is placed in the recess to separate the upper resilient element and the lower resilient element and to provide a limit of compression of the upper resilient element.

A limit of travel of the inner housing into the bore is selected. Consequently, a compressive force urging the inner housing to move relative to the outer housing from an unstressed configuration into the bore causes compression of the upper resilient element and the lower resilient element such that the upper resilient element and the lower resilient resist the compressive force and urge the inner housing back to the unstressed configuration.

A clip is attached to the inner housing such that the inner housing slides axially within the bore relative to the clip and the clip rotates in unison with the inner housing within the bore between the inner housing and the outer housing. In some embodiments, a lip in the outer housing and the cap constrains the clip from translating axially with respect to the outer housing. A second resilient element is placed within the bore between the inner housing and the outer housing. The second resilient element includes a first resilient member and a second resilient member that extend between the clip and the protrusion to resist rotational movement of the inner housing relative to the outer housing. Consequently, a torsional force that urges relative rotation between the inner housing and the outer housing from an aligned or unrotated configuration of the inner housing and the outer housing causes compression of the second resilient element such that the second resilient element resists the torsional force and urges the inner housing and outer housing back to the aligned configuration.

Other objects and advantages of the disclosed shock absorber system for a prosthesis will be apparent from the following description, the accompanying drawings, and the appended claims.
Fig. 1 is a perspective view of an embodiment of the shock absorber system for a prosthesis, shown attached to a prosthetic foot;
Fig. 2 is a side elevation of the shock absorber system of Fig. 1;
Fig. 3 is a side elevation in section of the shock absorber system taken at line 3-3 of Fig. 2;
Fig. 4 is an exploded view of the shock absorber system and prosthetic foot of Fig. 1;
Fig. 5 is a side elevation of the inner housing of the shock absorber system of Fig. 1, shown in an uncompressed state;
Fig. 6 is a side elevation in section of the shock absorber system taken at line 6-6 of Fig. 5;
Fig. 7 is a side elevation of the inner housing of the shock absorber system of Fig. 1, shown partially compressed;
Fig. 8 is a side elevation in section of the shock absorber system taken at line 8-8 of Fig. 7;
Fig. 9 is a side elevation of the inner housing of the shock absorber system of Fig. 1, shown fully compressed;
Fig. 10 is a side elevation in section of the shock absorber system taken at line 10-10 of Fig. 9;
Fig. 11 is a graph of force versus deflection for an embodiment of the shock absorber system of Fig. 1, showing two spring constants;
Fig. 12 is a graph showing stiffness versus deflection for the embodiment of Fig. 11;
Fig. 13 is a side elevation of another embodiment of the disclosed shock absorber system for a prosthesis adapted to be used as an add-on adapter;
Fig. 14 is a side elevation in section of the shock absorber system taken at line 14-14 of Fig. 13;
Fig. 15 is a side elevation in section of another embodiment of the disclosed shock absorber system for a prosthesis, shown attached to a prosthetic foot;
Fig. 16 is a cross section of the shock absorber system taken at line 16-16 of Fig. 15;
Fig. 17 is a side elevation in section of the embodiment of Fig. 15;
Fig. 18 is a front elevation in section of the embodiment of Fig. 15;
Fig. 19 is a detail of the inner housing of the embodiment of Fig. 15;
Fig. 20 is a detail of the clip of the embodiment of Fig. 15;
Fig. 21 is a detail of the assembly of the inner housing, clip and, first and second resilient members of the embodiment of Fig. 15;
Fig. 22 is a cross section of the shock absorber system taken at line 22-22 of Fig. 18 in which the inner housing is in an aligned configuration;
Fig. 23 is the cross section of the shock absorber system of Fig. 22 in which the inner housing is rotated from the aligned configuration with the outer housing shown in Fig. 22; and
Fig. 24 is a side elevation in section of another embodiment of the disclosed shock absorber system.

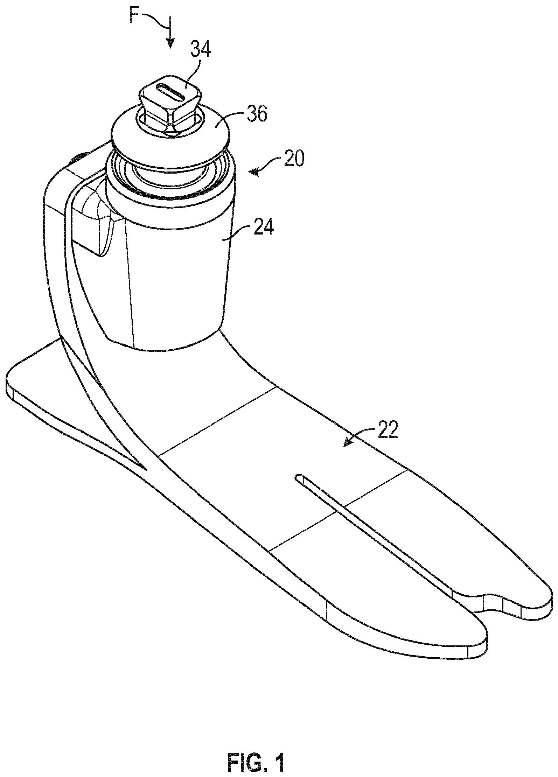

As shown in Figs. 1, 2, 3, and 4, an embodiment of the shock absorber system for a prosthesis, generally designated 20, is shown attached to a prosthetic foot 22. Prosthetic foot 22 in an embodiment may have a split toe and a heel plate and may be made of carbon fiber reinforced plastic (CFRP) or fiber glass reinforced plastic. The shock absorber system 20 includes an outer housing 24, which may be made of stainless steel, aluminum, or alloys of these, having a bore 26. An inner housing 28, which also may be made of stainless steel, aluminum, or alloys of these, is received within the bore 26 for slidable movement of the inner housing relative to the outer housing 24. An upper resilient element 30 is received within the inner housing 28 and resists movement of the inner housing into the bore 26. A lower resilient element 32 is received within the inner housing 28 that also resists movement of the inner housing into the bore 26. As will be discussed in greater detail, a compressive force F urging the inner housing 28 to move relative to the outer housing 24 from an unstressed or extended configuration shown in Figs. 1-3 into the bore 26 causes compression of the upper resilient element 30 and the lower resilient element 32 that is proportional to the magnitude of the compressive force such that the upper resilient element and the lower resilient resist the compressive force and urge the inner housing back to the unstressed configuration.

In an embodiment, the inner housing 28 includes a first fitting 34 and a concave dome 36 that covers and protects the top of the outer housing 24 and the bore 26. In an embodiment, the first fitting 34 takes the form of the male pyramid connector shown that attaches to a prosthetic socket (not shown), such as a female pyramid connector or other adapter. In an embodiment, the outer housing 24 includes a second fitting 38 that attaches to the prosthetic foot 22 such that when attached the shock absorber system 20 interconnects the prosthetic socket and the prosthetic foot. In an embodiment, the second fitting 38 includes mounting screws 40 and a shim 42. The screws 40 pass through holes 44 in a vertical segment 46 of the prosthetic foot 22 and holes 48 in the shim 42 and thread into threaded holes (not shown) formed in the side of the outer housing 24. The shim 42 is shaped to provide a flat seating surface for the screws 40 against the vertical segment 46 of the prosthetic foot 22. Alternatively, the outer housing 24 is attached to the prosthetic foot 22 by bonding, such as by a suitable adhesive.

When the male pyramid connector 34 of the inner housing 28 is attached to a prosthetic socket, such as by engaging a female pyramid connector (not shown), and the second fitting 38 attaches the shock absorber system 20 to the prosthetic foot 22, the weight of the user that is placed on the residual limb inserted in the prosthetic socket is transmitted through the prosthetic socket through the shock absorber system 20 and to the prosthetic foot 22, which is on a support surface (not shown). This weight transmission exerts the compressive force F on the shock absorber system 20 that causes the inner housing 28 to be displaced into the bore 26 of the outer housing 24. This displacement is resisted by the upper resilient element 30 and the lower resilient element 32.

In an embodiment, the shock absorber system includes a stop, generally designated 50, separating the upper resilient element 30 and the lower resilient element 32. The stop 50, which in embodiments is made of a rigid plastic or a nylon, limits compression of the upper resilient element 30 in response to the compressive force F that forces the inner housing 28 into bore 26 of the outer housing 24. In an embodiment, the stop 50 includes a central travel limiter 52 positioned between the upper resilient element 30 and the lower resilient element 32. In an embodiment, the stop 50 includes an end travel limiter 54 positioned at an end 56 of the upper resilient element 30 opposite the central travel limiter 52.

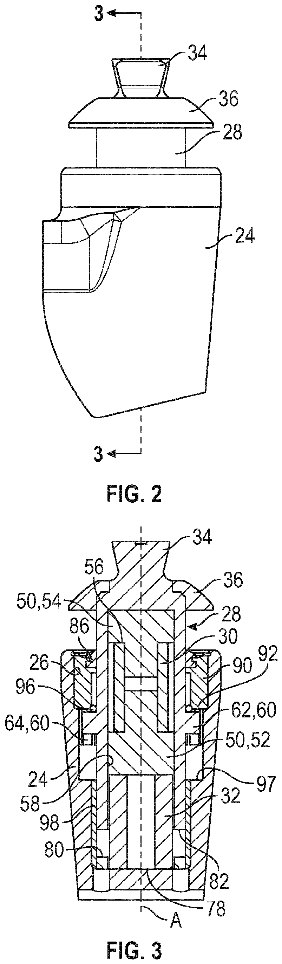

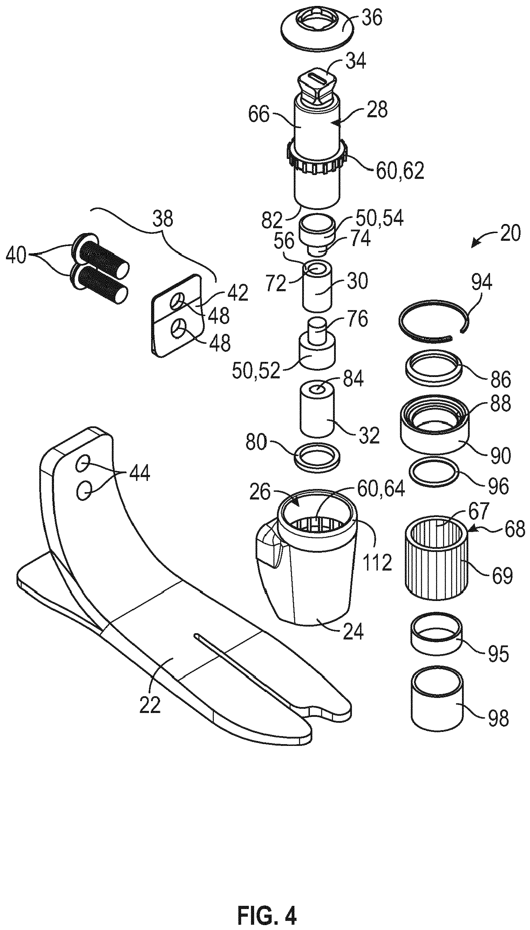

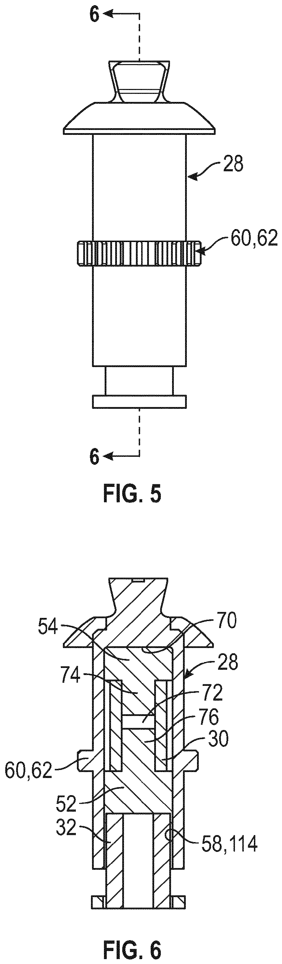

In an embodiment, the inner housing 28 includes a recess 58 (see also Fig. 6) that receives the upper resilient element 30, the lower resilient element 32, the end travel limiter 54, and the central travel limiter 52. In an embodiment, the inner housing 28 moves relative to the outer housing 24 along a central axis A of the bore 26. In an embodiment, shown for example in Figs. 3 and 4, a splined engagement, generally designated 60, between the inner housing 28 and the outer housing 24 prevents relative rotation between the outer housing and the inner housing about the central axis A. In an embodiment, the splined engagement 60 takes the form of raised ribs or splines 62 extending radially from an outer surface 66 of the inner housing 28 that engage and slide within longitudinal grooves 67 formed in the inner wall of a cylindrical spline bearing 68. Cylindrical spline bearing 68 includes longitudinal grooves 69 on its outer surface that engage longitudinal ribs 64 formed in the inner wall defining the bore 26.

In an embodiment, the inner housing 28 and the bore 26 of the outer housing 24 are cylindrical in shape such that the inner housing telescopes into the outer housing. The recess 58 in the inner housing 28 is cylindrical in shape and the upper resilient element 30 and the lower resilient element 32 are cylindrical in shape and fit within the recess. The end travel limiter 54 abuts an upper end 70 of the recess. The upper resilient element 30 is captured between the end travel limiter 54 and the central travel limiter 52.

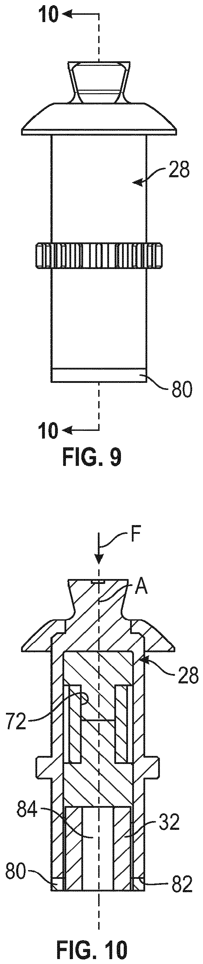

At least one of the central travel limiter 52 and the end travel limiter 54 includes a boss that extends partially into a passage 72 through the upper resilient element and engages the other of the end travel limiter or the central travel limiter to define a compression limit of the upper resilient element. In a particular embodiment, the end travel limiter 54 includes a boss 74 and the central travel limiter includes a boss 76, both of which extend into the passage 72 of the resilient element. In an embodiment, the outer housing 24 includes a bottom 78 at the bottom of the bore 26. A high impact travel limiter 80, which in embodiments is annular in shape, is mounted in the bottom 78 and engages an open end 82 of the inner housing 28 when the inner housing is fully displaced into the bore 26 (Figs. 3 and 6), as shown in Figs. 9 and 10.

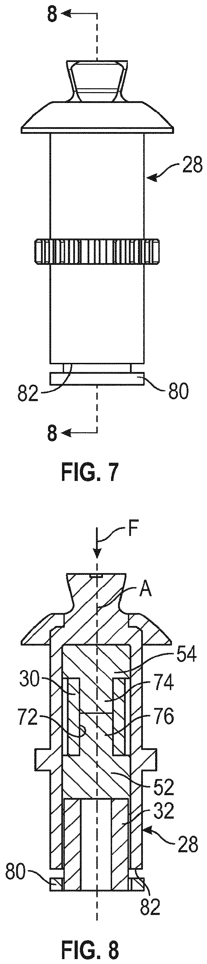

As shown in Fig. 1, for example, displacement of the inner housing 28 from its fully extended position into the recess 58 of the outer housing occurs when the weight of a user or wearer is applied to the associated prosthesis and is transmitted through the shock absorber system 20 to the prosthetic foot 22. As shown in Figs. 3 and 6, in response to this weight force F, the inner housing 28 is displaced further into the bore or recess 58 of the outer housing 24, as shown in Figs. 8 and 10. This displacement is resisted initially by a combination of the upper resilient element 30 and the lower resilient element 32. As shown in Figs. 7 and 8, as the weight or compressive force F applied increases, the bosses 74, 76 of the end travel limiter and the central travel limiter, respectively, meet in the passage 72 and abut each other, which effectively bottoms out the upper resilient element 30 by preventing its further compression within the recess 58 as the inner housing 28 continues to move into the bore 26 as a result of application of the compressive force F.

In an embodiment, at this point the open end 82 of the inner housing 28 has not contacted the high impact travel limiter 80 so that continued displacement of the inner housing into the bore 26 is still possible and the lower resilient element 32 is not yet fully compressed. Accordingly, the thickness of high impact travel limiter 80 is selected to limit travel of the inner housing 28 into the bore 26 a predetermined distance, thereby limiting an amount of compression of the lower resilient element 32 before bottoming out, as shown in Figs. 9 and 10. Alternatively, the length of the inner housing 28 compared to the height of the lower resilient element 32 and/or the thicknesses of the end and central travel limiters 52, 54, respectively is selected to define the travel of the inner housing into and out of the bore 26. In embodiments, the lower resilient element 32 includes a central passage 84 to allow for expansion of the lower resilient element 32 in response to compression from the displacement of the inner housing 28 into the bore 26 of the outer housing 24.

In embodiments, the upper resilient element 30 and the lower resilient element 32 are made of an elastomer, such as natural or synthetic rubber, or a urethane. Alternatively, the upper and lower resilient elements 30, 32, respectively, take the form of coil springs. In still other embodiments, the upper and lower resilient elements 30, 32 are a combination of an elastomer and a coil spring. In embodiments, the end and central travel limiters 52, 54, respectively, are made of a comparatively less compressible material than the upper and lower resilient elements, 30, 32, respectively, such as a metal, hard rubber, or a hard plastic such as nylon.

In an embodiment, the upper resilient element 30 has a first spring constant k1 and the lower resilient element 32 has a second spring constant k2. Application of the compressive force F displacing the inner housing 28 into the bore 26 first compresses both the upper resilient element 30 and the lower resilient element 32 resulting in a combined spring constant kT of the first spring constant k1 and the second spring constant k2 resisting the compressive force F for a first displacement interval until the upper resilient element reaches a predetermined fully compressed state (Figs. 7 and 8). Then, further compression beyond the predetermined fully compressed state of the upper resilient element 30 for a second displacement interval results in the lower resilient element 32 alone resisting the displacing of the inner housing 28 into the bore 26 at the second spring constant k2 (Figs. 9 and 10) in response to the force F, at which point the inner housing 28 is fully displaced into the bore 26.

According to Hooke's law, the combined spring constant kT provided by the upper resilient element 30 and the lower resilient element 32 is kT = (1/k1 + 1 /k2)-1. In some embodiments, the spring constants k1 and k2 are selected such that the second spring constant is greater than the first spring constant: k1 < k2 (or, alternatively, the reverse). With such an embodiment, in response to a relatively small value for the compressive force F, which would occur during ambulatory activity such as normal walking, mostly the upper resilient element 30 is compressed. The difference in amount of compression between the upper resilient element and the lower resilient element 32 would depend upon the magnitude of the difference between selected spring constants k1 and k2. The upper resilient element 30 and the lower resilient element 32 are therefore selected so that kT provides optimal cushioning comfort to the user for walking, considering such variables as the normal gait and weight of the user. The spring constant k2 of the lower resilient element 32 is selected for optimal cushioning comfort for a relatively large value of the compressive force F, which would occur during higher ambulatory activity such as running and jumping.

In still other embodiments the spring constant values k1 and k2 of the first and lower resilient elements 30, 32, respectively, are the same, or approximately the same, since, according to Hooke's law supra, the combined spring constant kT would be less than either k1 or k2 alone to provide optimal cushioning for low impact ambulatory activity such as walking. In these embodiments, in which one spring constant is either the same or greater than the other, the length of one or both bosses 74, 76 is adjusted to modulate the degree of compression of the upper resilient element 30 and thus the transition from the first displacement interval to the second displacement interval in response to the compressive force F.

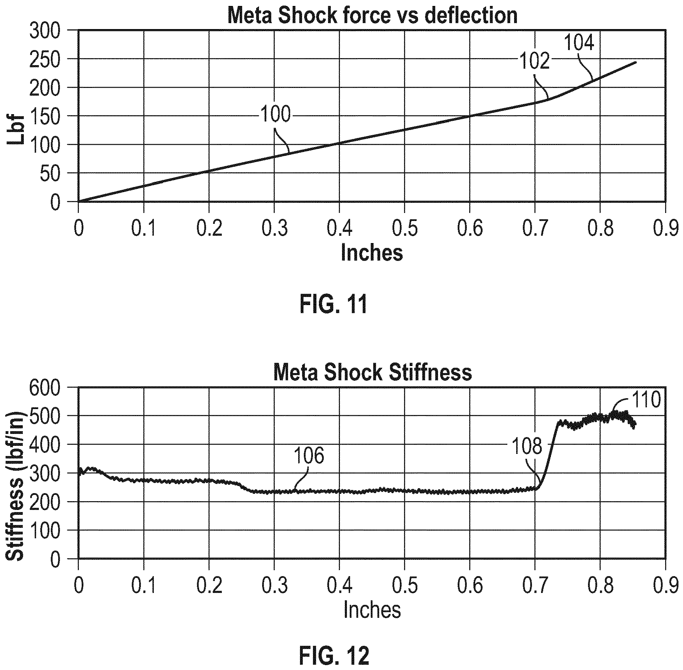

As shown in Fig. 11 for an exemplary embodiment of the shock absorber system 20, compressive force F in units of foot-pounds is plotted against displacement x in units of inches. The first displacement interval, represented by line segment 100, is a straight line of a first slope corresponding (approximately) to kT in an embodiment in which the first spring constant k1 is less than the second spring constant k2, or in the embodiment in which the first and second spring constants are approximately equal. The inflection or transition point 102 is where the upper resilient element 30 bottoms out, as shown in Figs. 7 and 8. The second displacement interval 104 is represented by a line segment of a second, steeper slope corresponding (approximately) to spring constant k2. This shows that the second displacement interval 104 requires a greater compressive force F for each incremental displacement of the inner housing 28 into the bore 26 of the outer housing 24 to accommodate relatively higher stress ambulatory activities.

As shown in Fig. 12, stiffness of a shock absorber system 20 embodiment in units of foot-pounds per inch is plotted against fractional inches of displacement of the inner housing 28 into the bore 26 of the outer housing 24. The line segment 106 represents the stiffness of the shock absorber system 20 during first displacement interval in which a compressive force F of approximately 300 foot-pounds per inch displaces the inner housing 28 into the bore 26 until the inflection or transition point 108 is reached, when the spring constant changes from kT to k2, shown by the second displacement interval, represented by line segment 110. At that point a greater compressive force F is required, in this embodiment of about 1722 Nm/cm (500 ftlb/in.), for additional displacement.

An exemplary embodiment of the shock absorber system 20 is shown in Figs. 3 and 4. An annular wiper gasket 86 slidably engages the outer surface 66 of the inner housing 28 and is received within an annular recess 88 in an upper retaining cap 90. The cap 90, which in embodiments is an upper retaining cap, is seated on a shoulder 92 formed in the bore 26 and is retained in place by a split or snap ring 94. The upper retaining cap 90 includes a radially inner undercut that receives an upper sleeve bearing 95 that slidably engages the outer surface 66 of the inner housing 28 and is located above the splined engagement 60 as shown in Fig. 3. The O-ring 96 that in embodiments is seated on the outer surface 66 and above the splined engagement 60 of the inner housing 28. A lower sleeve bearing 98 is seated on the bottom 78 of the bore 26 radially outside the high impact travel limiter 80 and slidably receives the inner housing 28. The space in the bore 26 between the top of the lower sleeve bearing 98 and the bottom of the upper sleeve bearing 95 houses the spline bearing 68 and allows travel for the splines 62 on the inner housing 28 along axis A.

In an exemplary embodiment, the splined engagement 60 includes the spline bearing 68 that is seated on an annular shoulder 97 formed in the inner surface of the bore 26. The spline bearing 68 is retained on the annular shoulder 97 by the lower end of the cap 90. The spline bearing 68 interconnects the inner housing 28 to the outer housing 24 in a non-rotating engagement about axis A through the engagement of the splines 62 with the longitudinal grooves 67 and the longitudinal grooves 69 with the longitudinal ribs 64.

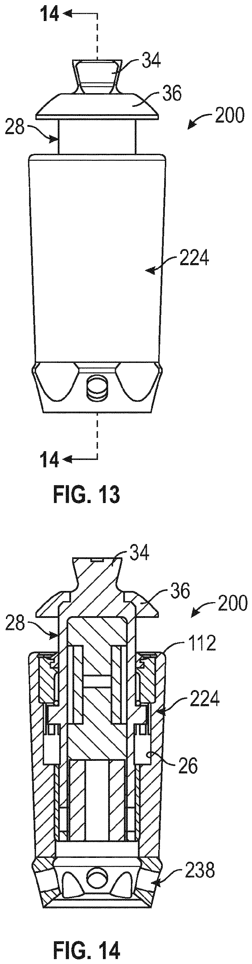

As shown in Figs. 13 and 14, a shock absorber system 200 is disclosed for use as an add-on adapter for a prosthesis, for example a prosthetic limb such as a leg (not shown). The shock absorber system includes a male pyramid connector 34 and concave dome 36. The inner housing 28 is structurally the same as inner housing 28 shown in Figs. 3 and 6. The outer housing 224 includes a bore 26 that also is structurally the same as bore 26 of Figs. 3 and 6. However, the outer housing 224 includes a fitting in the form of a female pyramid adapter 238. In embodiments, the male pyramid connector 34 may releasably attach to the distal connector, such as a female pyramid adapter, of the socket of a prosthetic component, such as a pylon, knee, socket, or the like. The female pyramid adapter 238 may releasably attach to the proximal component, such as a male pyramid adapter, of the proximal component of a prosthetic lower limb or prosthetic foot.

In a one exemplary embodiment, shock absorber system 20, 200 for a prosthesis includes an outer housing 24, 224 attachable to a prosthetic foot 22 or limb (not shown). The outer housing 24 includes bore 26 with an upper, proximal opening 112 and a bottom 78 (Figs. 3 and 4) and a distal or second fitting 38, 238 that attaches to a prosthesis. An inner housing 28 is attachable to a prosthetic socket and is received within the bore 26 for slidable movement of the inner housing along a central axis A of the bore. The inner housing 28 has a cylindrical wall 114 defining a recess 58 with a proximal upper end 70, an open lower, distal open end 82, and a proximal fitting including a male pyramid connector 34 that attaches to a prosthetic socket assembly.

The upper resilient element 30 takes the form of a first cylindrical resilient element 30 that is received within the recess 58 and resists movement of the inner housing 28 into the bore 26. The lower resilient element 32 takes the form of a second cylindrical resilient element 32 that is received within the recess 58 and resists movement of the inner housing 28 into the bore 26. An end travel limiter 54 is positioned in the recess 58 abutting the upper end 70 and an upper end of the first cylindrical resilient element 30. A central travel limiter 52 is positioned in the recess 58 and abuts a lower end of the first cylindrical resilient element 30 and an upper end of the second cylindrical resilient element 32 (as shown in Figs. 3, 6, 8, and 10), thereby separating the first cylindrical resilient element from the second cylindrical resilient element. Thus, the first cylindrical resilient element 30 is captured between and abuts the end travel limiter 54 and the central travel limiter 52 and the lower resilient element 32 is captured between and abuts the central travel limiter and the bottom 78 of the cylindrical bore.

One or both of the end traveler limiter 54 and the central travel limiter 52 include a boss 74, 76 extending at least partially through the passage 72 of the first cylindrical resilient element 30 such that engagement of the end traveler limiter and the central travel limiter defines a limit of compression of the first cylindrical resilient element from displacement of the inner housing 28 into the cylindrical bore 26. A high impact travel limiter 80 abuts the bottom 78 of the cylindrical bore 26 and is positioned to engage the lower open distal end 82 of the cylindrical wall of the inner housing 28, thereby defining a limit of compression of the second cylindrical resilient element 32 from displacement of the inner housing into the cylindrical bore.

A compressive force F urging the inner housing 28 to move relative to, and in embodiments into the bore 26 of, the outer housing 24 from an unstressed, fully extended configuration (Figs. 3, 6, and 14) into the bore 26 causes compression of the first cylindrical resilient element 30 and the second cylindrical resilient element 32, as shown in Figs. 7-10. The first cylindrical resilient element 30 and the second cylindrical resilient element 32 resist the compressive force F and urge the inner housing 28 back toward the unstressed, fully extended configuration shown in Figs. 5 and 6. In an embodiment, the first cylindrical resilient element 30 has a spring constant k1 that is less than a spring constant k2 of the second cylindrical resilient element 32.

It should be noted that, in use, the inner housing 28 typically will be in a partially extended state in which the inner housing is displaced from its fully extended configuration into the bore 26 of the outer housing 24. This partial compression or partial extension results from the normal force of weight of the wearer bearing down on the inner housing 28 through the male pyramid connector 34 and transmitted to the prosthetic foot 22. When the wearer places their full weight on the shock absorber system 20, 200, such as during running or stair climbing, the inner housing 28 is displaced into the bore 26 in a relatively compressed state.

In an exemplary embodiment of a method of making a shock absorber system, such as the shock absorber system 20, for a prosthesis to interconnect a prosthetic foot 22 or limb with a prosthetic socket, a bore 26, which in embodiments is a cylindrical bore, is formed in an outer housing 24 and a cylindrical recess or bore 58 is formed in an inner housing 28. The inner housing 28 is placed within the bore 26 for slidable movement of the inner housing relative to the outer housing 24. An upper resilient element 30 is placed within the recess 58 of the inner housing 28 to resist movement of the inner housing into the bore 26. A lower resilient element 32 is placed within the recess 58 of the inner housing 28 to resist movement of the inner housing into the bore 26.

A central travel limiter 52 is placed in the recess 58 to separate the upper resilient element 30 and the lower resilient element 32 and to provide a limit of compression of the upper resilient element. A limit of travel of the inner housing 28 into the bore 26 is selected, whereby a compressive force F urging the inner housing to move relative to the outer housing 24 from an unstressed configuration into the bore causes compression of the upper resilient element 30 and the lower resilient element 32 such that the upper resilient element and the lower resilient resist the compressive force and urge the inner housing back to the unstressed configuration.

In an embodiment, the method further includes selecting the upper resilient element 30 and the lower resilient element 32 to have a combined spring constant kT appropriate for a user of a given weight to walk. And in an embodiment, the method further includes selecting the lower resilient element 32 to have a second spring constant k2 appropriate for the user to jump.

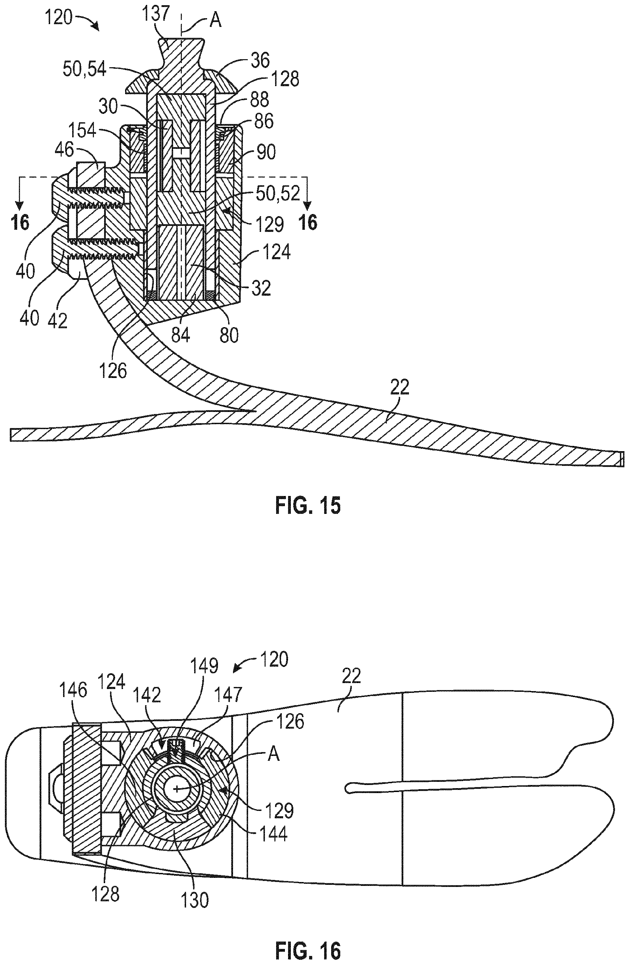

As shown in Figs. 15, 16, and 17, another embodiment of the disclosed shock absorber system for a prosthesis, generally designated 120, accommodates and cushions torsional loads as well as vertical loads. In Figs. 15-17, the components having the same reference numbers as the components shown in the embodiments of Figs. 1-14 and discussed supra, have the same structure and function. As with the shock absorber system 20, 200 of the embodiments of Figs. 1-14, the shock absorber system 120 in an exemplary embodiment is attachable to a prosthesis, such as the prosthetic foot 22. The shock absorber system 120 includes an outer housing 124 having a bore 126. In embodiments, the outer housing is made of stainless steel, aluminum, or metal alloys.

An inner housing 128, which in embodiments is made of stainless steel, aluminum, or metal alloys, is received within the upper (when attached to prosthetic foot 22) open end of a bore 126. The inner housing 128 is mounted in the bore 126 for relative rotational movement between the inner housing and the outer housing 124, and in embodiments, relative movement linearly along central axis A of the inner housing and bore of the outer housing. The structure and function of the inner housing 128, and of the upper resilient element 30, lower resilient element 32, stop 50 including central travel limiter 52 and end travel limiter 54, high impact travel limiter 80, and open end 82 are as shown in Figs. 3 and 4-10 and described in the corresponding text.

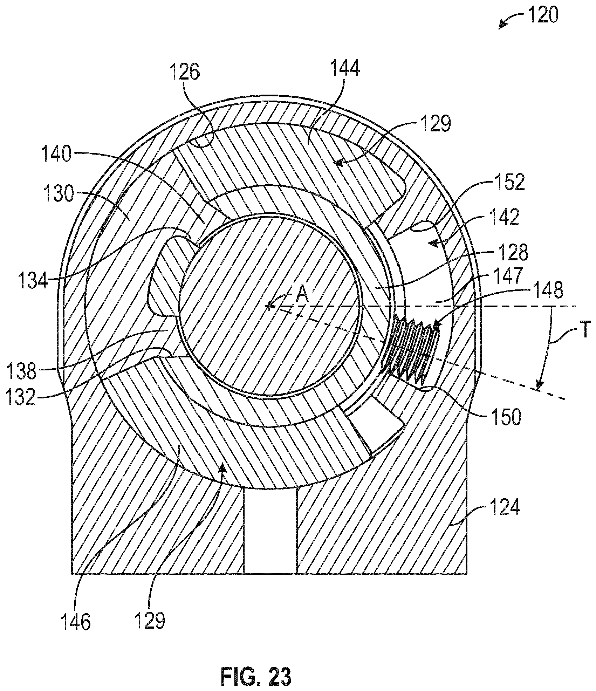

A resilient element, generally designated 129, is received within the bore 126 of the outer housing 124. The second resilient element 129 resists the relative rotational movement between the inner housing 128 and the outer housing 124. Such rotational movement may occur when a wearer of the prosthetic foot 22 places their weight on the prosthetic foot 22 and pivots about the foot by pivoting a prosthetic socket (not shown) attached to the male pyramid adapter 137 extending upwardly from the inner housing 128. The resulting torsional force causes relative rotational movement between the inner housing 128 and the outer housing 124 from an aligned configuration of the inner housing and the outer housing, shown for example in Fig. 16, to a pivoted configuration shown for example in Fig. 23. This relative rotational movement by the inner housing 128 causes compression of the second resilient element 129 such that the resilient element resists the torsional force and urges the inner housing and outer housing back to the aligned configuration.

In an embodiment, the outer housing 124 is adapted to be attached to the prosthetic foot 22 by screws 40 that extend through a shim 42 and thread through the vertical segment 46 of the of the prosthetic foot 22. In an embodiment the shock absorber system 120 interconnects a prosthetic socket, which it engages with male pyramid adapter 137, with the prosthetic foot 22.

In an embodiment, the second resilient element 129 is positioned within the bore 126 between the inner housing 128 and the outer housing 124. As shown in Fig. 16, in an embodiment, the second resilient element 129 is arcuate in shape and contacts the cylindrical body 136 of the inner housing 128 and a surface of the outer housing 124 defining a wall of the bore 126. In a particular embodiment, the bore 126, second resilient element 129, and inner housing 128 are concentric with axis A, so that the inner housing rotates clockwise and counterclockwise about axis A relative to the outer housing 124 (see Figs. 16, 22, and 23).

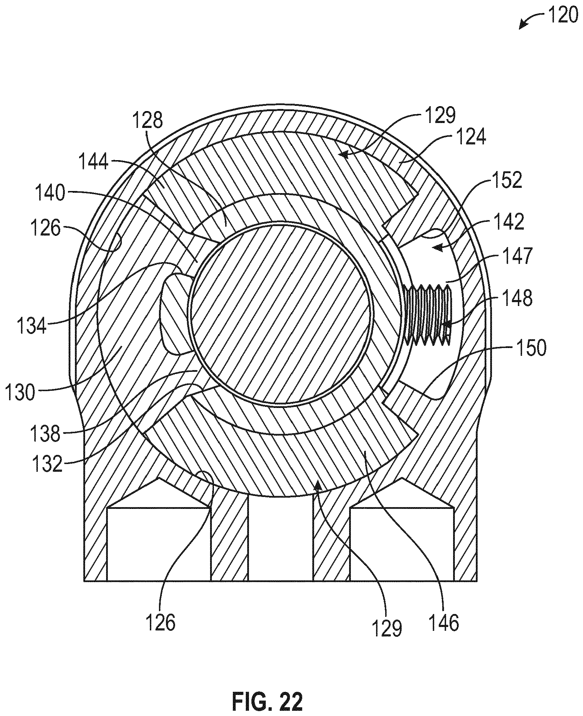

As shown in Figs. 16, 18, and 20, in an embodiment, a clip 130 is positioned between the inner housing 128 and the outer housing 124 within the bore 126, such that the clip is restrained from axial movement along axis A relative to the outer housing and slidably engages the inner housing during the axial movement of the inner housing. The clip 130 is arcuately shaped to conform to the curvature of the wall of the bore 126 and abutting body 136 of the inner housing 128 to slidably engage the outer housing 124 for relative rotational movement between the outer housing and the inner housing. The clip 130 engages the inner housing 128 to rotate with the inner housing. Torsional movement between the inner housing 128 and the outer housing 124 from the aligned configuration (Figs. 16 and 22) to the rotated configuration (Fig. 23) causes the clip 130 to compress the second resilient element 129. In embodiments, the clip 130 may be made of metal, such as aluminum, brass, stainless steel, or alloys, or of a hard plastic, such as nylon or acetal, or of a hard rubber, such as nitrile rubber.

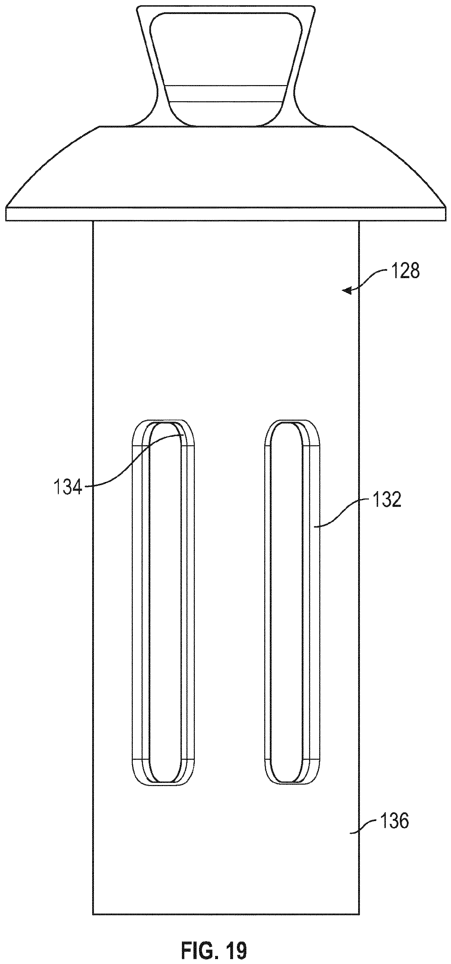

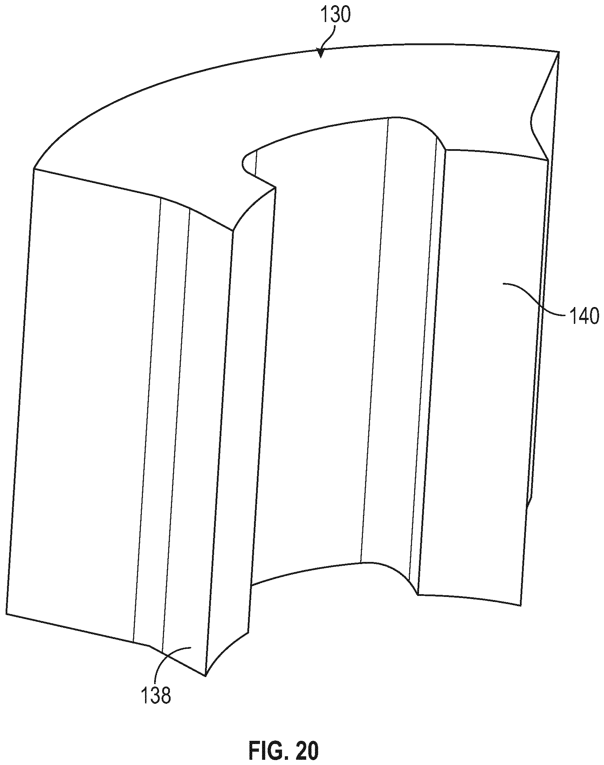

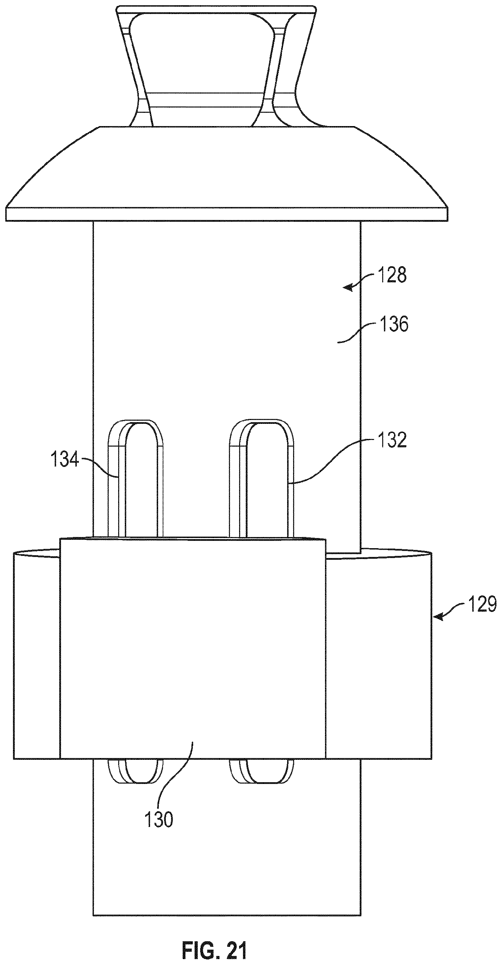

In an embodiment, as shown in Fig. 19, the inner housing 128 includes at least one elongate slot 132, and in embodiments a second, parallel elongate slot 134, extending along the cylindrical body 136 of the inner housing in an axial direction. In embodiments, as shown in Fig. 20, the clip 130 includes at least one rib 138, and in embodiments a second rib 140, shaped to extend into the elongate slots 132, 134, respectively, as shown in Figs. 22 and 23. Engagement of the ribs 138, 140 with the corresponding elongate slots 132, 134 permits relative axial movement of the inner housing 128 and the clip 130 and constrains the clip to rotate with the inner housing during the relative torsional movement between the inner housing and the outer housing 124. In an embodiment, the clip 130 is shaped to engage and abut the second resilient element 129, as shown in Figs. 16, 21, 22, and 23.

As shown in Figs. 16, 17, 18, and 22, in an embodiment, the outer housing 124 includes an arcuate protrusion 142 extending into the bore 126 from, and is fixed relative to, the outer housing 124 and that abuts the second resilient element 129. The second resilient element 129 is positioned between the protrusion 142 and the clip 130, whereby the torsional movement T between the inner housing 128 and the outer housing 124 causes the second resilient element 129 to be compressed between the clip and the protrusion. In embodiments, the protrusion 142 is unitary with the outer housing 124. In other embodiments, the protrusion 142 is a separate piece that is attached by a suitable adhesive, brazing, welding, or fasteners.

In an exemplary embodiment, the second resilient element 129 includes a first resilient member 144 and a second resilient member 146. The first resilient member 144 and the second resilient member 146 each are arcuate in shape and extend between the clip 130 and the protrusion 142 to resist the torsional force that results in the torsional movement T of Fig. 23 and urge the inner housing and outer housing back to the aligned configuration of Fig. 22.

In an embodiment, the protrusion 142 includes an arcuate recess 146, and the inner housing includes a projection 148 that extends into the recess. The recess 146 is bounded by radially inward extending and axially extending walls 150, 152. The walls 150, 152 define limits of relative rotational travel between the inner housing 128 and the outer housing 124. The limit of relative rotational travel in a given direction is designated by T in Fig. 23 for relative rotation in a clockwise direction. It is to be understood that in the embodiment shown there is a complementary limit of relative rotation in a counterclockwise direction when the shock absorber system 120 is viewed from above, as shown in Figs. 16, 22, and 23 that is equal or approximately equal in magnitude.

In embodiments, the projection 148 is selected from a boss extending radially from an outer surface of the cylindrical body 136 of the inner housing 128 and a set screw extending radially from the outer surface of the inner housing. In an embodiment, the projection 148 takes the form of a pair of set screws. In an embodiment, the recess 146 is elongate in an axial direction and is shaped to permit displacement of the projection 148 within the recess during relative axial movement between the inner housing 128 and the outer housing 124 along axis A as the inner housing moves linearly relative to the outer housing between an extended or fully extended position within the bore 126 and a relatively compressed position within the bore.

As shown in Figs. 15, 17, and 18, in an embodiment, a cap 90 is retained within the bore 126 and engages the inner housing 128 and outer housing 124. The cap 90 permits the relative axial movement and the relative rotational movement between the inner housing 128 and outer housing 124. In embodiments, the cap 90 is cylindrical in shape and includes an upper sleeve bearing or bushing 154 to reduce friction between the cylindrical body 136 of the inner housing 128 and the cap 90. In an embodiment, a lower end (i.e., further within the bore 126) of the cap 90 is shaped to abut the upper ends (i.e., shallower within the bore 126) of the walls 150, 152 of the protrusion 142 and of the clip 130. Thus, the cap 90 is partially seated on the protrusion 142 within the bore 126. In embodiments, there is enough clearance between the lower end of the cap 90 and the clip 130 to allow the clip to rotate relative to the cap and outer housing 124 with rotation of the inner housing 128.

In an embodiment, the bore 126 includes an annular, radially inward projecting ledge 156. The first and second resilient members 144, 146 of the second resilient element 129 are captured between the ledge 156 at a lower end of the bore and the cap 90 at an upper edge of the bore. The ledge 156 and the cap 90 constrain movement of the second resilient element 129 in an axial direction within the bore. The cap 90 constrains the upward movement of the projection 148 within the bore 126 and hence provides a limit of travel or full extension of the inner housing 128 out of the bore. In an embodiment, the bore 126 includes a lower sleeve bearing or bushing 159 that provides a low-friction engagement with the outer surface of the cylindrical body 136 of the inner housing 128 below the second resilient element 129.

As described with reference to the embodiment of the shock absorber system 20 illustrated in Figs. 1-10, 13, and 14, in embodiments, the cap 90 is retained within the bore 126 by a snap ring 94. Alternatively, or in addition, the cap 90 is retained within the bore 126 by a press fit, by a suitable adhesive, fasteners such as screws (not shown), and/or by welding or brazing. In certain embodiments, it may be desirable to removably attach the cap 90 within the bore 126, in which case the cap is retained only by the snap ring 94. In embodiments, the cap 90 includes an undercut that receives an annular wiper gasket 86, made of a flexible material such as nylon or rubber, that engages the cylindrical body 136 to prevent contaminants from entering the bore 126. In an exemplary embodiment, the high impact travel limiter 80 is annular in shape and is mounted in the bottom of the bore 126 to engage the lower end of the cylindrical body 136 and provide a hard stop for displacement of the inner housing 128 into the bore of the outer housing 124, which would denote a fully compressed configuration for the shock absorber system 120.

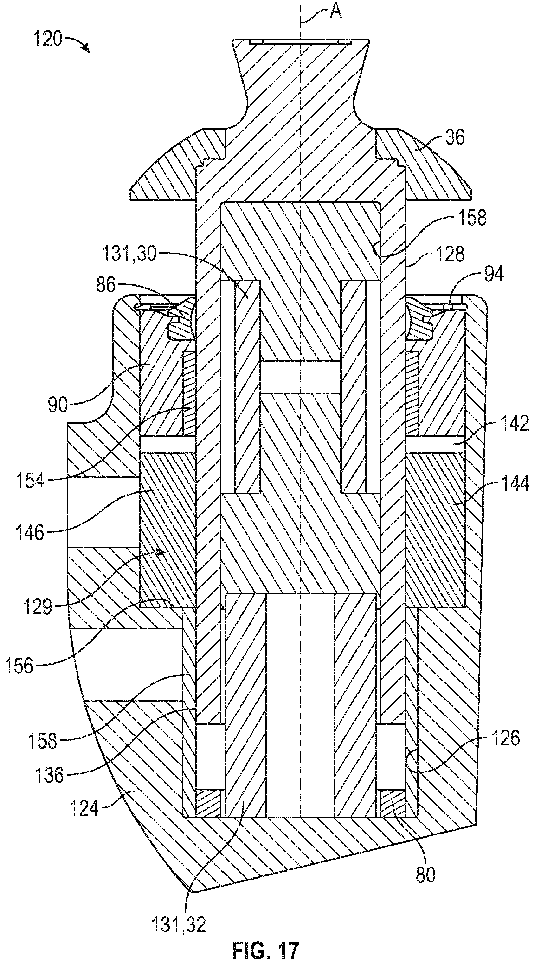

In a particular exemplary embodiment shown in Figs. 15, 17, 18, and 24, the shock absorber system 120 includes an outer housing 124 having a bore 126 and an inner housing 128 received within the bore for axial and rotational movement relative to the outer housing. A first resilient element, generally designated 131, is received within the outer housing 124 and resists the axial movement of the inner housing 128 into the bore 126 along axis A. A compressive force that urges the inner housing 128 to move from an uncompressed, fully extended, or relatively extended configuration, depending upon whether there is an initial loading on the inner housing, as shown for example in Figs. 17, 18, and 24, further into the bore 126 causes compression of the first resilient element 131. The first resilient element 131 resists the compressive force and urges the inner housing 128 back to the uncompressed or relatively extended configuration. This compression in response to the compressive force is the same as the compression of the inner housing 28 against the travel limiter in response to the compressive force F shown in Figs. 8 and 10.

In this particular embodiment, a second resilient element 129 that takes the form of a second resilient element received within the outer housing 124 and resists the rotational movement of the inner housing 128 relative to the outer housing. A torsional force, also represented by arrow T in Fig. 23, that urges relative rotation between the inner housing and the outer housing about axis A from the aligned configuration of the inner housing and the outer housing, shown for example in Figs. 16 and 22, causes compression of the second resilient element 129, and in particular, the second resilient member 146 in Fig. 23. The second resilient member 146 resists the torsional force T.

When the torsional force is released or removed, such as when a wearer of the system 120 and prosthetic foot 22 lifts the foot from the ground, the opposing resilient force exerted by the second resilient member 144 urges the inner housing to rotate relative to the outer housing back to the aligned configuration shown, for example, in Fig. 22, in which the foot faces forward and is aligned with the male pyramid connector 137 in a forward direction. Similarly, a torsional force exerted on the shock absorber system 120 in the opposite or counterclockwise direction from the force T shown in Fig. 23 causes compression of first resilient member 144, which exerts a reactive and restorative force to causes relative rotation between the inner housing 128 and outer housing 124 to the aligned configuration.

In the embodiment shown in Figs. 1, 16, and 22, when in the aligned configuration, the male pyramid connector 137 on the inner housing 128 is oriented relative to the prosthetic foot 22 such that the prosthetic foot faces forward when the connector is attached to a prosthetic socket worn by a user. In this configuration, the first resilient member 144 and the second resilient member 146, whose resilient forces oppose each other, are uncompressed, or in the alternative, each only slightly or relatively compressed by the same or approximately the same amount in a circumferential direction. In an embodiment, the spring constants of the first resilient element 131 and the second resilient element 129 in response to compression in a circumferential direction are the same or are approximately the same.

The balance of circumferential forces exerted by the first resilient member 144 and the second resilient member 146 against each other and through the clip 130 keep the prosthetic foot 22 facing forward and in an aligned configuration. In an embodiment, the projection 148 is centered within the recess 146 between the walls 150, 152 of the protrusion 142 in the bore 126 outer housing 124 when the shock absorber system 120 is in an aligned configuration, as shown for example in Fig. 22. This is achieved by positioning the projection 148 diametrically opposite the clip 130 on the cylindrical body 136 and sizing the first resilient member 144 and the second resilient member 146 to be equal, or substantially equal, in size, shape, and composition, and making the circumferential distances between the clip 130 and the recess 146 defined by walls 150, 152 the same or approximately the same.

This orientation allows maximum relative rotation between the outer housing 124 and inner housing 128 in a clockwise and a counterclockwise direction relative to Fig. 22. In other embodiments, the recess 146 is shaped and positioned such that the recess 146 within the protrusion 142 is selected to be off center when the shock absorber system 120 is in the aligned configuration of Fig. 16, which would permit greater rotation in one direction (e.g., rotating the prosthetic foot 22 outward, for example) than in the opposite direction (e.g., rotating the prosthetic foot inward, for example).

Summarizing, the shock absorber system 120 is adapted for use with a prosthesis, and in an exemplary embodiment interconnects a prosthetic foot 22 with a prosthetic socket, such as shown in commonly owned U.S. application Serial No. 16/356,499 titled Prosthetic Socket Sealing System and Method, the disclosure of which is incorporated herein by reference. The shock absorber system 120 includes an outer housing 124 having a bore 126 with a longitudinal axis A, a protrusion 142 extending radially into the bore, and an annular ledge 156 in the bore. An inner housing 128 is received within the bore for linear movement along the longitudinal axis A and relative rotational movement about the longitudinal axis between the inner housing 128 and the outer housing 124. The inner housing 128 has a cylindrical body 136 that includes a pair of elongate slots 132, 134 extending in a direction of the longitudinal axis A.

A clip 130 is mounted within the bore 126 between the inner housing 128 and the outer housing 124. The clip 130 is fixed within the bore 126 to resist axial movement relative to the outer housing 124. The clip 130 includes a pair of ribs 138, 140 that engage the elongate slots 132, 134 such that the inner housing 128 slides relative to the clip during the linear movement and the clip and the inner housing rotate in unison during the rotational movement. The clip 130 engages the outer housing 124 to restrict linear movement along the longitudinal axis. A cap 90 is mounted in the bore 126 and is attached to the outer housing 124. The cap 90 abuts the clip 130 and the protrusion 142 such that the clip is captured between the cap and the ledge 156 so that movement of the clip along the longitudinal axis A is restricted.

A first resilient element 131 including an upper resilient member 30 and a lower resilient member 32 is received within the inner housing 128. The first resilient element 131 resists movement of the inner housing 128 along the longitudinal axis A into the bore 126, wherein a compressive force F that urges the inner housing to move from a relatively uncompressed and extended configuration further into the bore to a relatively compressed and less extended configuration causes compression of the first resilient element between the inner housing and the outer housing such that the first resilient element resists the compressive force and urges the inner housing back to the uncompressed configuration.

A second resilient element 129 includes a first resilient member 144 and a second resilient member 146 mounted in the bore 126 between the inner housing 128 and the outer housing 124. The first resilient member 144 and the second resilient member 146 extend between the clip 130 and the protrusion 142 on opposite sides of the cylindrical body 136 of the inner housing 128. The first resilient member 144 and the second resilient member 146 are constrained from movement along the longitudinal axis A by the ledge 156 and the cap 90. The first resilient member 144 and the second resilient member 146 resist the relative rotational movement between the inner housing 128 and the outer housing 124 such that a torsional force T that urges the relative rotation from an aligned configuration of the inner housing and the outer housing causes compression of one of the first resilient member and the second resilient member such that the second resilient element resists the torsional force and urges the inner housing and outer housing back to the aligned configuration.

A method of making the shock absorber system 120 for a prosthetic foot 22 to interconnect a prosthetic foot or limb with a prosthetic socket is as follows. A bore 126 is formed in an outer housing 124 and a protrusion 142 is formed to extend into the bore. A recess 158 is formed in the inner housing 128. The inner housing 128 is placed within the bore 126 for axial and rotational movement relative to the outer housing 124.

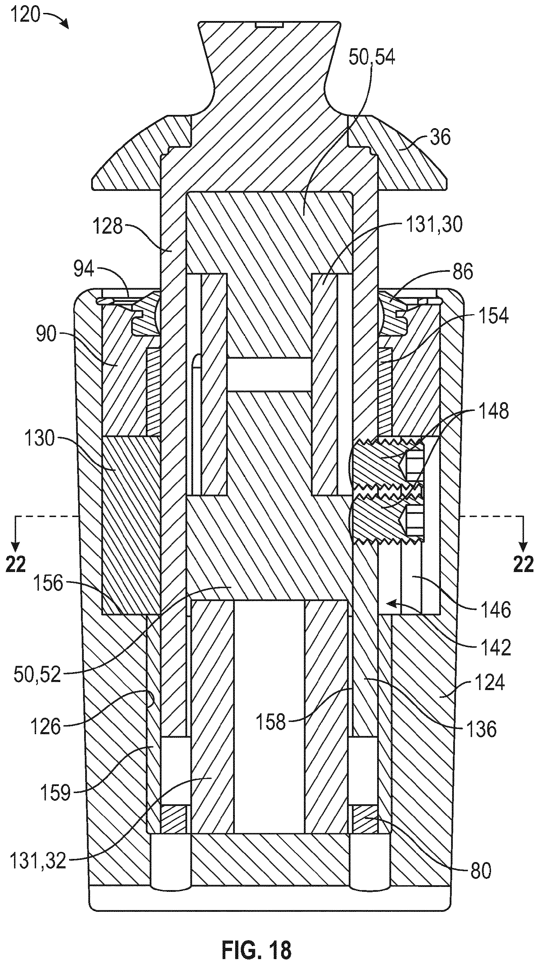

A first resilient element 131 is placed within the recess 158 of the inner housing 128. In an embodiment, this first resilient element 131 takes the form of placing an upper resilient member 30 and a lower resilient member 32 within the inner housing 128 to resist movement of the inner housing into the bore 126. A central travel limiter or stop 50 (see Figs. 4 and 18) is placed in the recess 158 to separate the upper resilient element 30 and the lower resilient element 32 and to provide a limit of compression of the upper resilient element.

A limit of travel of the inner housing 128 into the bore 126 is selected whereby a compressive force F urging the inner housing to move relative to the outer housing 124 from an unstressed configuration into the bore causes compression of the upper resilient element and the lower resilient element such that the upper resilient element and the lower resilient resist the compressive force and urge the inner housing back to the unstressed configuration. A clip 130 is attached to the inner housing 128 such that the inner housing slides axially within the bore 126 relative to the clip and rotates in unison with the inner housing within the bore between the inner housing and the outer housing 124.

A second resilient element 129 is positioned within the bore 126 between the inner housing 128 and the outer housing 124. This positioning of the second resilient element 129 includes placing a first resilient member 144 and a second resilient member 146 that extend between the clip 130 and the protrusion 142 to resist the rotational movement of the inner housing 128 relative to the outer housing124. Consequently, a torsional force F that urges relative rotation between the inner housing 128 and the outer housing 124 from an aligned configuration of the inner housing and the outer housing causes compression of the second resilient element 129 such that the second resilient element resists the torsional force and urges the inner housing and outer housing back to the aligned configuration.

In an embodiment, this method further includes placing the first and second resilient members 144, 146 within the bore 126 on opposite sides of the cylindrical body 136 of the inner housing 128. In embodiments, the method also includes press fitting a cap 90 within the bore 126 such that the clip 130 and the first and second resilient members 144, 146 are restricted in axial movement within the bore by the ledge 156 and the cap 90. In an embodiment, the method further includes forming a recess 147 in the protrusion 142 and forming a projection 148 that extends from the cylindrical body 136 of the inner housing 128 into the recess such that the recess defines a limit of relative rotational travel between the inner housing and the outer housing. In embodiments, the method includes selecting the projection 148 is selected from a boss extending radially from an outer surface of the inner housing and set screws 149 extending radially from the cylindrical body 136 of the inner housing.

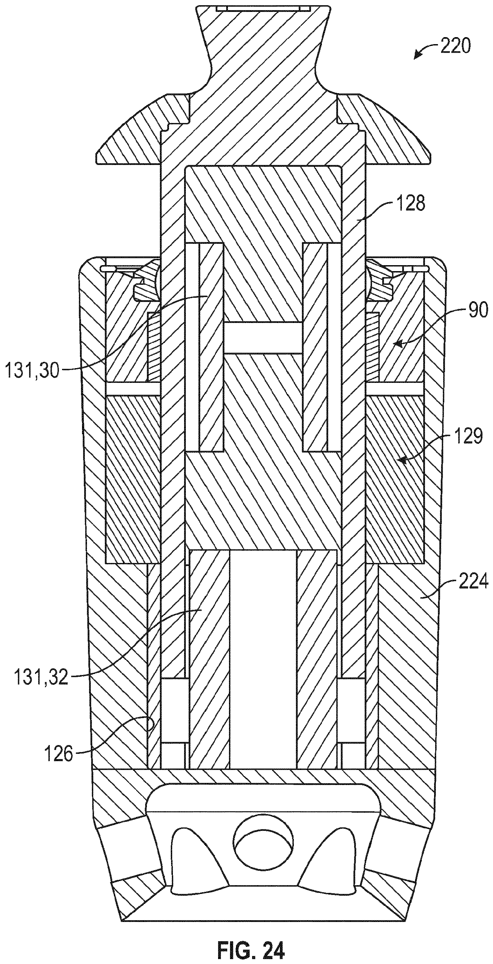

As shown in Fig. 24 an embodiment of the shock absorber system, generally designated 220, is identical to the embodiment 120 shown in Figs. 15-23 in all respects except for the structure of the outer housing, generally designated 224. The outer housing 224 is adapted to interconnect to a prosthetic socket (not shown) with a prosthetic component other than the prosthetic foot 22, and functions as an add-on adapter for a prosthesis, for example a prosthetic limb such as a leg (not shown). The outer housing 224 terminates at a lower end in a fitting such as a female pyramid adapter, and in this respect is similar to housing 224 of Figs. 13 and 14. Accordingly, the disclosed shock absorber system 120, 220 is adaptable to a wide variety of prosthetic applications.

The disclosed shock absorber systems 20, 120, 200, 220 have the advantages of being relatively low cost and lightweight yet provide spring resistance and shock absorption that is ideal for a wide range of ambulatory movement, from a relatively low stress force from activity such as walking to a relatively high stress force from an activity such as running or jumping. While the forms of apparatus and methods disclosed constitute preferred embodiments of the disclosed shock absorber system it is to be understood that the scope of the invention is not limited to these precise apparatuses and methods and that changes may be made therein without departing from the scope of the invention.

## Claims

1. A shock absorber system (20) for a prosthesis, the shock absorber system **characterized by**:
an outer housing (24) having a bore (26);
an inner housing (28) received within the bore for slidable movement of the inner housing relative to the outer housing;
an upper resilient element (30) received within the inner housing that resists movement of the inner housing into the bore; and
a lower resilient element (32) received within the inner housing that resists movement of the inner housing into the bore;
whereby a compressive force (F) urging the inner housing (28) to move relative to the outer housing (24) from a relatively extended configuration into the bore (26) causes compression of the upper resilient element (30) and the lower resilient element (32) such that the upper resilient element and the lower resilient element resist the compressive force and urge the inner housing back to the relatively extended configuration.

2. The shock absorber system (20) as claimed in claim 1, wherein the inner housing (28) includes a first fitting (34) that attaches to a prosthetic socket, wherein the first fitting includes a male pyramid connector.

3. The shock absorber system (20) as claimed in claims 1 or 2, wherein the outer housing (24) includes a second fitting (38) that attaches to a prosthetic limb or foot (22) such that when attached, the shock absorber system interconnects the prosthetic socket and the prosthetic foot or limb.

4. The shock absorber system (20) as claimed in any of claims 1-3, further **characterized in that** a stop (50) separates the upper resilient element (30) and the lower resilient element (32); wherein the stop limits compression of the upper resilient element in response to the compressive force (F).

5. The shock absorber system (20) as claimed in claim 4, wherein the stop includes a central travel limiter (52) positioned between the upper resilient element (30) and the lower resilient element (32).

6. The shock absorber system (20) as claimed in claim 5, wherein the stop (50) includes an end travel limiter (54) positioned at an end of the upper resilient element (30) opposite the central travel limiter (52).

7. The shock absorber system (20) as claimed in any of claims 5 or 6, wherein the inner housing (28) includes a recess (58) that receives the upper resilient element (30), the lower resilient element (32), the end travel limiter (54), and the central travel limiter (52).

8. The shock absorber system (20) as claimed in any of claims 1-7, wherein the inner housing (28) moves relative to the outer housing (24) along a central axis (A) of the bore (26); and the shock absorber system is further **characterized by** a splined engagement (60) between the inner housing (28) and the outer housing (24) that prevents relative rotation between the outer housing and the inner housing about the central axis.

9. The shock absorber system (20) as claimed in any of the preceding claims, wherein the upper resilient element (30) has a first spring constant (k1) and the lower resilient element (32) has a second spring constant (k2) different from the first spring constant; whereby application of the compressive force (F) displacing the inner housing (28) into the bore (26) first compresses both the upper resilient element (30) and the lower resilient element (32) resulting in a combined spring constant (kT) of the first spring constant and the second spring constant resisting the compressive force until the upper resilient element reaches a predetermined compressed state, then further compression results in the lower resilient element alone resisting the displacing of the inner housing into the bore at the second spring constant.

10. The shock absorber system (200) as claimed any of the preceding claims, **characterized in that**:
the inner housing (128) is mounted within the bore (126) for relative rotational movement in response to a torsional force between the inner housing and the outer housing (124); and
a resilient element (129) is received within the outer housing (124) that resists the relative rotational movement such that a torsional force that urges the rotational movement from an aligned configuration of the inner housing and the outer housing causes compression of the resilient element wherein the resilient element resists the torsional force and urges the inner housing and outer housing back to the aligned configuration.

11. The shock absorber system (200) as claimed in claim 10, wherein the outer housing (124) is adapted to be attached to a prosthetic limb, and the inner housing (128) includes an adaptor for attachment to a prosthetic socket.

12. The shock absorber system (200) as claimed in claim 11, wherein the resilient element (129) is positioned between the inner housing (128) and the outer housing (124).

13. The shock absorber system (200) as claimed in claim 12, **characterized in that**
a clip (130) is positioned between the inner housing (128) and the outer housing (124), wherein the clip is restrained from axial movement relative to the outer housing and slidably engages the inner housing during the axial movement of the inner housing, and the clip slidably engages the outer housing for relative rotational movement and engages the inner housing to rotate with the inner housing; rotational movement between the inner housing and the outer housing from the aligned configuration causes the clip to compress the resilient element (129);
the inner housing includes at least one slot (132) extending in an axial direction;
the clip (130) includes at least one rib (138) extending into the slot (132); whereby engagement of the at least one rib with the at least one slot permits relative axial movement of the inner housing (128) and the clip and constrains the clip to rotate with the inner housing during the relative rotational movement between the inner housing and the outer housing; and
wherein the clip engages the resilient element.

14. The shock absorber system (200) as clamed in claim 13, wherein the outer housing (124) includes a protrusion (142) extending into the bore (126) and abutting the resilient element (129); and the resilient element is positioned between the protrusion (142) and the clip (130), whereby the torsional movement between the inner housing (128) and the outer housing (124) causes the resilient element to be compressed between the clip and the at least one rib (138).

15. A shock absorber system (200) for a prosthesis, **characterized by**:
an outer housing (124) having a bore (126);
an inner housing (128) received within the bore for axial and rotational movement relative to the outer housing;
a first resilient element (131) received within the outer housing (124) that resists the axial movement of the inner housing into the bore, wherein a compressive force (F) that urges the inner housing to move from a relatively extended configuration further into the bore causes compression of the first resilient element such that the first resilient element resists the compressive force and urges the inner housing back to the relatively extended configuration; and
a second resilient element (129) received within the outer housing (124) that resists the rotational movement of the inner housing (128) relative to the outer housing, wherein a torsional force that urges relative rotation between the inner housing and the outer housing from an aligned configuration of the inner housing and the outer housing causes compression of the second resilient element such that the second resilient element resists the torsional force and urges the inner housing and outer housing back to the aligned configuration.
